# EUROPEAN PATENT APPLICATION

(11) **EP 4 763 486 A1**
(43) Date of publication of application: **24.06.2026**
(21) Application number: 24221594.5
(22) Date of filing: 19.12.2024
(51) Int. Cl.: B29C 70/42, B29C 70/54

(54) **CURE AND FLOW FRONT DETECTION SYSTEM AND ASSOCIATED DEVICES**

(71) Applicant: Airbus S.A.S., 31700 Blagnac (FR)
(72) Inventor: Buchmann, Christopher, 82024 Taufkirchen (DE); Sayeh, Mohamed, 82024 Taufkirchen (DE); Vogel, Daniel, 82024 Taufkirchen (DE); Springer, Maximilian, 82024 Taufkirchen (DE); Le Gleuher, Celine, 82024 Taufkirchen (DE); Karunanithi, Kannan, 82024 Taufkirchen (DE)
(74) Representative: KASTEL Patentanwälte PartG mbB

(57) **Abstract**

In order to improve molding processes for composite parts, the invention proposes a system for detecting a cure and/or flow front in a molding apparatus. The system comprises a mold portion, a sensor apparatus (22), and a control unit. The mold portion has a molding surface (14) that is configured to contact a preform (16) to be infused with resin. The sensor apparatus (22) is arranged within the mold portion and includes a sensor element (24) that extends in a longitudinal direction. The sensor element (24) is arranged to be at least partially below the molding surface (14) into the mold portion such that the sensor element (24) is capable of interaction with resin and/or the preform (16), e.g. via electric fields. The control unit is configured to control the sensor element (24), so as to detect a cure and/or flow front based on a change in the interaction, such as the electric field, due to the flowing or curing of resin.

## Description

The invention relates to monitoring of composite manufacturing processes. In particular the invention relates to a system for detecting cure and flow front in a molding apparatus or process. Furthermore, the invention relates to feedthrough arrangements that are used for connecting sensors within the molding apparatus to the outside.

EP 3 035 041 A1 and EP 3 035 042 A2 disclose devices and methods for cure and flow front detection using time-domain reflectometry.

It is the object of the invention to improve monitoring of cure and flow front (CFF) detecting systems.

The invention provides a system for detecting a cure and/or flow front in a molding apparatus, the system comprising:
- a mold portion having a molding surface that is configured to contact a preform to be infused with resin;
- a sensor apparatus is arranged within the mold portion and includes a sensor element that extends in a longitudinal direction and is arranged to be at least partially below the molding surface into the mold portion such that the sensor element is capable of interaction with resin and/or the preform; and
- a control unit that is configured to control the sensor element, so as to detect a cure and/or flow front based on a change in the interaction due to the flowing or curing of resin.

Preferably, the sensor element is configured for generating an electric field that is capable of interacting with the resin and/or the preform. Preferably, the control unit is configured to supply to and to receive from the sensor element electrical energy, so as to detect a cure and/or flow front based on a change of the electrical field due to the flowing or curing of resin.

Preferably, the sensor element includes a dielectric and at least one signal wire embedded in the dielectric, wherein the signal wire is arranged such that, when the signal wire generates an electrical field, the electrical field is capable of interacting with resin and/or the preform.

Preferably, the sensor element includes an odd number of signal wires and the control unit is configured to cause generation of an electrical field between pairs of adjacent signal wires.

Preferably, the mold portion includes an accommodation groove that accommodates and engages the sensor apparatus or the sensor element, and the accommodation groove is open towards the preform.

Preferably, the accommodation groove includes at least one retaining feature that retains the sensor apparatus or the sensor element, in a direction orthogonal to the molding surface. Preferably, the sensor apparatus or the sensor element include at least one retaining member that is detachably fixed to the mold portion and/or the molding surface, so as to prevent the sensor apparatus and/or the sensor element from moving out of the accommodation groove under its own weight.

Preferably, the sensor apparatus comprises a sealing member that is fixed, preferably detachably fixed, to the mold portion and that is arranged to seal the sensor element from the preform in order to prevent resin flow along the longitudinal direction of the sensor apparatus.

Preferably, the sensor apparatus comprises a lid member that includes a second accommodation groove and that is attached to the mold portion or to another member, and the sensor element is accommodated in the second accommodation groove.

Preferably, a lid sealing member is arranged between the lid member and the mold. Preferably, the lid member has a thinned portion adjacent to the second accommodation groove, the thinned portion being configured so as to allow interaction of the sensor element with resin or the preform.

Preferably, the sensor apparatus further comprises a core member that includes a third accommodation groove and is the another member to which the lid member is attached. Preferably, the second and third accommodation grooves form an accommodation channel that accommodates the sensor element.

Preferably, the depth of the second accommodation groove is greater than the depth of the third accommodation groove so that the sensor element is closer to the molding surface in an orthogonal direction relative to the molding surface.

Preferably, the mold portion includes a feedthrough recess that is configured to allow feedthrough of a connecting cable to the sensor element.

The invention provides a molding apparatus for molding a preform into a finished part by introducing resin into the preform, the molding apparatus comprising a previously described system and a mold that has the mold portion.

The invention provides a molding arrangement occurring during a molding process in a preferred molding apparatus, the molding arrangement comprising a preform or a resin infused preform arranged on the molding surface, wherein the control unit controls the sensor element to so as to detect the preform and/or resin in the vicinity of the sensor element.

The invention provides a method for detecting a cure and/or flow front in a molding process using a previously described system, molding apparatus or molding arrangement, the method comprising:
- at least partially arranging a sensor apparatus that has a sensor element that extends in a longitudinal direction within a mold portion and arranging the sensor element to be at least partially below the molding surface into the mold portion such that the sensor element is capable of interacting with resin and/or the preform;
- with a control unit controlling the sensor element for detecting a cure and/or flow front based on a change in the interaction due to the flowing or curing of resin.

The invention provides a feedthrough arrangement for feedthrough of an electrical or optical connection from the outside to a sensor element within a mold that is suitable for resin introduction into a preform, the feedthrough arrangement comprising a mold portion that includes a through hole and a feedthrough member that has a feedthrough passage and that is threadable into a threaded mounting hole so that the through hole and the feedthrough passage are aligned.

Preferably, the threaded mounting hole is formed in the mold portion so as to be aligned with the through hole and the feedthrough passage.

Preferably, the threaded mounting hole and/or the feedthrough member define a resin chamber for resin to ingress into.

Preferably, the feedthrough arrangement includes a mounting plate that is attachable to the mold, so as to form a planar contact with the mold, and includes the threaded mounting hole.

Preferably, the mounting plate is attached with threaded fasteners. Preferably, the mounting plate includes a sealing groove and a sealing element arranged therein, so that the sealing element is pressed against the mold, when the mounting plate is attached.

Preferably, the threaded mounting hole is a conical thread and the feedthrough member includes a matching threaded portion.

The invention provides a feedthrough arrangement for feedthrough of an electrical or optical connection from the outside to a sensor element within a multi-part mold that is suitable for resin introduction into a preform, the feedthrough arrangement comprising a first mold portion that includes a feedthrough passage and a second mold portion that includes an accommodation groove for the sensor element, wherein, when the multi-part mold is in the closed state, the feedthrough passage and the accommodation groove are aligned so that a feedthrough cable is capable of contacting the sensor element.

Preferably, the first mold portion includes a mold protrusion protruding from the first mold portion and the second mold portion includes a sealing groove that is capable of accommodating the mold protrusion, when the multi-part mold is closed.

Preferably, the sealing groove includes a sealing element that is arranged to sealingly engage the mold protrusion, when the multi-part mold is closed. Preferably, the sealing element is arranged on the bottom of the sealing groove so that the mold protrusion sealingly engages with its outermost surface.

Preferably, the feedthrough passage includes a feedthrough cable that is permanently fixed in the feedthrough passage, and the feedthrough cable protrudes from the first mold portion so as to be capable of operatively connecting to a sensor element arranged in the accommodation groove.

Preferably, the second mold portion includes a sensor element that is arranged in the accommodation groove, so as to be capable of operatively connecting to a feedthrough cable that is arrange within the feedthrough passage.

Preferably, the feedthrough arrangement includes a locking mechanism that is configured to detachably lock the feedthrough cable and the sensor element together, when the multi-part mold is closed, and to disengage, when the multi-part mold is opened.

The ideas disclosed herein focus on facilitating the integration of line sensors or cables into molds/tools for serial production processes such as Resin Transfer Molding (RTM) of composite parts, especially for aircraft. When utilizing line sensors, e.g. CFF sensors or fiber-optic sensors, to monitor the production process, the sensor has been integrated directly into the part. However, integrating sensors the part is not ideal. Especially in the composite sector, the stability of the parts may be improved by getting rid of integrated line sensors in order to reduce complexity of design and costs.

The idea aims to both increase the reusability of the line sensors as well as to decrease their effect onto the part and process (e.g. vacuum infusion or RTM). By integrating the sensor into the mold, its effect on the part is reduced. Furthermore, the cost of per part reduces due the sensor remaining in the mold and being utilized for several parts rather than just one. It should be noted that while the description focuses on dielectric sensors, the ideas described herein also apply for other line sensors types such as fiber-optic sensors.

Another idea is a special CFF sensor design (basically a modified coaxial cable) and a groove in a mold for holding the modified cable as the sensor in place. A standard coax cable comprises one or multiple signal wires embedded in a dielectric (e.g. PTFE). The dielectric is surrounded by a shielding and an insulating layer. A coax cable can be used as CFF sensor in the invention, when the insulation and the shielding are removed. Since the dielectric will be in contact with the composite fibers and the resin in the mold, the dielectric should be qualified for this purpose, which is a given with the usual PTFE dielectric.

The sensor is inserted into a specifically designed groove within the mold. The groove may in general have an arbitrary cross section, but is adapted to the cross section of the sensor (here: circular as the modified coax cable has a circular cross section). The sensor is retained at an offset from the molding surface of the mold, i.e. below the molding surface and within the mold. This allows a small undercut which retains the sensor securely in place, even if the sensor is placed overhead, for example. A small fillet radius may simplify the placement of the sensor and decrease the risk of damage to the dielectric medium. By varying the dimensions (radius, fillet radius and offset) the imprint of the protruding section of the sensor above the molding surface and onto the composite laminate can be adjusted. The coefficient of thermal expansion of the dielectric (e.g. PTFE) is roughly one to two orders of magnitude higher than the mold material (usually steel or invar). Thus the dielectric expands further than the groove at the elevated temperatures during the RTM process, for example, which closes any gaps that may exists between the sensor and the mold, thereby preventing resin from leaking into the groove. If significant movement of resin along gaps between preform and mold, also called race tracking, is still occurring near the small fillet radius, the groove and sensor can be covered by a thin strip of adhesive tape. This concept cannot only be used with a CFF sensor, but also for other line sensors, such as fiber-optic sensors, or cables.

A further idea is based on the preceding concept, but instead of modifying a coax cable, a CFF sensor with custom cross section is produced. Any number of cables (one signal and two ground for the CFF sensor) are embedded in a suitable dielectric (e.g. PTFE) with an arbitrary cross section (e.g. rectangular). A rectangular cross section is easier to handle and the required groove is less difficult and thus less expensive to machine. Other non-primitive geometry with advantages in terms of handling and securing may be utilized as well, but require a more complex to machine groove. Again, any gaps between groove and dielectric will be closed due to the different thermal expansion of dielectric and mold at elevated process temperatures. The sensor can be flush with the surface of the mold to minimize race tracking and imprints. RTM-qualified tape can also be utilized to keep the sensor in place in case it must be mounted overhead.

Another non-primitive geometries for the dielectric may feature nibs as retainers that prevent the dielectric from falling out of the groove in situations where the mold is moved around.

It is also possible to cover the sensor with a thin lid made out of metal (preferably same material as the mold) which allows the whole setup to be more durable and the mold to be cleaned more easily. Any variant of the before mentioned CFF sensors may be attached to the lid and mounted into the tooling as one part. The thickness of the lid near the sensor is small enough to allow detection of the resin above it. Tight tolerances and an additional sealing may reduce race tracking between mold and lid as well as ingress of resin into the cavity. This setup also allows to place the feedthrough for the sensor underneath the lid, preventing accumulations of resin in the proximity of the feedthrough.

Once sensor and lid are integrated into the RTM mold, it can be cleaned using advanced technologies such as hydro blasting, which would otherwise damage the sensors. This makes the whole sensor system more robust and suitable for serial production as the sensors do only have to be replaced in case of maintenance.

Similar to the previous idea, the sensor may not only be covered by a lid but also be embedded in a core element covered by the lid. The advantage of this concept is that the sensor cable is completely shielded from the environment even when not built into the mold. The whole assembly of sensor, core and lid is also mechanically more robust and easier to handle for workers who are usually already trained in inserting cores into the mold.

Another aspect of the invention relates to facilitating a data/power/signal-line from a (high) temperature/(high) pressure/vacuum environment (e.g. molds in RTM process) to an ambient environment in a serial production framework. One idea is to use existing feedthroughs. Those are used to feed data/power/signal cables, tubes or sensors into a process chamber with different environment than ambient. They can be directly mounted onto the outside of the mold by means of a conical thread.

While this thread has sealing capabilities and is useful, sometimes liquid resin may have direct contact with the feedthrough: The high pressure inherent to the RTM process, for example, may squeeze resin into the first few convolutions of the conical thread. Thus, the resin will harden within the thread, which can make it difficult or even impossible to dismount the feedthrough without damage to the thread. However, dismounting the feedthrough regularly is necessary for cleaning of the mold (removing resin) during serial production. Even if the thread of the feedthrough remains undamaged after dismounting, it needs to be cleaned in order to be reused another time. Removing hardened resin from a thread is a difficult and tedious process.

One idea aims to further reduce the required effort per part (e.g. cleaning time), while providing a connection of the data/power/signal line from ambient to process environment, as well as ensuring that both environments stay separated from each other.

With the improvements disclosed herein, it is especially possible to ensure repeated usability of feedthroughs, which are in contact with liquid mediums that cure directly at or in close proximity of the feedthrough within the process chamber (e.g. resin in RTM). With the suggestions herein, the feedthrough may be dismounted more easily. It can be reused multiple times and allows that the mold can be cleaned with less effort or with more advanced methods (e.g. hydro blasting). It is also possible to modularize the feedthroughs by aggregating them together in a group.

The feedthrough may be threaded into an adapter plate that may have a sealing mechanism (e.g. sealing cord). The plate is preferably fixed to the mold by bolts. The feedthrough is preferably permanently mounted to the adapter plate by means of its conical thread. Ingress of resin into the first convolutions of the conical thread may appear but are rather irrelevant since this connection need not be loosened in regular operation. Instead the whole assembly of feedthrough and adapter plate can be demounted. The sealing cord enables that no leak occurs between adapter plate and tool, so that this design reduces or avoids accumulations of cured resin on features that are typically loosened and tightened in regular operation (e.g. threads).

In addition, the geometry of the accumulations of cured resin in the space between adapter plate and tooling (flat plate shape) is easier to break up and can be cleaned easier than resin accumulations in a thread, as the whole adapter plate can be pried open.

In another variant, a sealing groove, an adapter cable/measuring cable with special geometry and a sealing cord are provided. The adapter cable is preferably permanently mounted to the upper tooling e.g. glued in the male part of the groove. Ingress of resin into a cavity between upper and lower mold may appear but is acceptable as the molds can be cleaned after an infusion by e.g. hydro blasting. The sealing cord restricts the resin to the "wet"-zone.

The sensor cable is preferably integrated in the lower mold. By closing the upper and lower mold together a connection between the adapter and the sensor cable is made. This connection allows the signal to be sent to the measuring unit.

The main advantage of this feedthrough is that this type does not need any new holes in the mold damaging/effecting the surface of the part. It also prevents leakage in the part cavity. The signal can be transferred via the sensor and adapter cable directly to the measuring unit.

This disclosure comprises a second invention that relates to monitoring of a composite manufacturing process, preferably for foreign objects. The second invention relates further to a system for detecting foreign objects during the composite manufacturing process.

Carbon fiber reinforced plastic parts can be automatically manufactured by automated fiber placement (AFP). The machine subsequently places layers of preimpregnated (prepreg) tows on top of each other to build the composite part.

Typically the prepreg tow is provided on spools, where thin foil separates each tow from the tow laying underneath in order to prevent them from sticking together. At a certain point within the lay-up machine the tows are separated from the thin foil. It is crucial for the manufacturing process that the thin foil, also sometimes called backing tape, does not get placed together with the tows onto the part, as this foreign object is typically diagnosed as a defect which may increase the risk of delamination. As a result, such parts would have to be repaired or scrapped.

This idea allows for an early detection of foreign objects with the possibility to interrupt the manufacturing process and remove the foreign objects either by hand or other means. After removal, the manufacturing process may be continued. It is also possible to detect foreign objects that are currently difficult to detect, as for example, when the foil stays on the side of the prepreg that will face downward when placed onto the part surface or tool surface. The sensor used may be small enough to be integrated into the machine while the machine does not lose any flexibility due to geometrical restrictions.

One idea is to have a visual sensor system that is capable of monitoring the prepreg tows after the removal of the foil and detecting the foil that possibly remains on the prepreg.

The sensor may be configured as a line scanner or an area camera. The sensor may be a CMOS-type, CCD-type, or a contact-type image sensor. At least one LED or other lighting source may be used for illuminating the monitored prepreg. The light source may be used for creating a better contrast between the tow and the foil. The captured image may be analyzed by typical image analysis methods/algorithms.

The second invention is defined in the following clauses.

### Clause 1:

A method for detecting foreign objects during a composite manufacturing process, the method comprising:
a) providing a fiber source that includes a plurality of layers of a fiber material and a separation member that is arranged between adjacent layers of the fiber material;
b) transporting the fiber material from the fiber source to a manufacturing location, at which a fiber composite part gets built;
c) with a sensor apparatus: scanning the fiber material during transporting in step b) for separation member residue of the separation member on the fiber material and/or a foreign object on the fiber material;
d) if separation member residue and/or the foreign object is detected in step c), generating an interruption signal that is adapted to interrupt the manufacturing process, in order to allow removal of the separation member residue and/or the foreign object.

### Clause 2:

The method according to clause 1, wherein the fiber source includes at least one fiber storage, each fiber storage having stored therein/thereon the layers of fiber material and the separation member, wherein step b) comprises transporting the fiber material from each fiber storage to the manufacturing location.

### Clause 3:

The method according to clause 2, wherein the fiber storage is a fiber storage spool on which the fiber material is wound in layers and adjacent layers are separated by the separation member.

### Clause 4:

The method according to clause 2 or 3, wherein step c) comprises scanning the fiber material being transported from each fiber storage with the sensor apparatus for separation member residue and/or the foreign object.

### Clause 5:

The method according to any of the clauses 1 to 4, wherein in step c) the sensor apparatus scans the fiber material by illuminating the fiber material with a light source.

### Clause 6:

The method according to clause 5, wherein the light source illuminates the fiber material with light that impinges with an angle of incidence from 0° to 10°on the fiber material, wherein the angle of incidence is measured relative to the normal on the fiber material.

### Clause 7:

The method according to any of the clauses 1 to 6, wherein the scanning apparatus comprises a scanning sensor that scans the fiber material during transporting, wherein the scanning sensor is chosen from a group consisting of a line scanning sensor and an area camera sensor.

### Clause 8:

The method according to any of the clauses 1 to 7, wherein the scanning apparatus comprises a scanning sensor that scans the fiber material during transporting, wherein the scanning sensor is chosen from a group consisting of a CMOS-type sensor, a CCD-type sensor, and contact-type sensor.

### Clause 9:

The method according to any of the clauses 7 or 8, wherein the scanning sensor is an imaging sensor.

### Clause 10:

A process for manufacturing a fiber composite part, preferably for an aircraft, the method comprising:
a) performing a method according to any of the clauses 1 to 9;
b) laying up the fiber material on a tool surface or a fiber material layer of the part;
c) interrupting step b) upon receipt of the interruption signal.

### Clause 11:

The method according to clause 10, further comprising the step:
d) removing the separation member residue and/or the foreign object from the fiber material.

### Clause 12:

The method according to any of the clauses 10 or 11, further comprising the step:
e) continuing with step b) or aborting the manufacturing process.

### Clause 13:

A manufacturing apparatus configured for manufacturing a fiber composite part, the manufacturing apparatus comprising:
- a fiber source that includes a plurality of layers of a fiber material and a separation member that is arranged between adjacent layers of the fiber material;
- a transport mechanism configured for transporting the fiber material from the fiber source to a manufacturing location, at which a fiber composite part gets built;
- a sensor apparatus configured for scanning the fiber material during transporting for separation member residue of the separation member on the fiber material and/or a foreign object on the fiber material;
- a control unit that is configured to perform a method according to any of the clauses 1 to 12.

### Clause 14:

The manufacturing apparatus according to claim 13, further comprising a fiber material placement head that is operatively connected to the fiber source via the transport mechanism in order to receive the fiber material, wherein the fiber material placement head is controlled by the control unit to lay up the fiber composite part using the fiber material, wherein the sensor apparatus is integrated into the fiber material placement head.

### Clause 15:

The manufacturing apparatus according to clause 13 or 14, wherein the sensor apparatus is arranged such that the fiber material is scanned before being laid up.

### Clause 16:

The manufacturing apparatus according to any of the clauses 13 to 15, wherein the sensor apparatus includes a non-reflective background, and the fiber material is arranged between the sensor apparatus, preferably the scanning sensor, and the non-reflective background.

With these ideas it is possible to detect separation member residue or other foreign objects on the fiber material, e.g. tow prepreg. The final part may have increase stability, due to the residue or foreign object being removed. It is possible to detect residue or foreign objects down to a size of about 5 mm by 5 mm. The idea is independent of the shape of the residue or the foreign objects. A computer vision algorithm, which is known per se, can be used to determine the presence of residue or foreign objects. Experiments using a line scanner and LED lights in front of a non-reflective surface have shown that a reliable detection of residue or foreign objects is possible for the desired size. Moreover, the experiments have shown that detection is possible for various speeds of up to 1 m/s. Furthermore, since the fiber material may vibrate, an out of focus distance of about 8 mm is non-detrimental to the detection accuracy. Overall it is therefore possible to improve manufacturing processes of composite materials by reducing the risk for defects, in particular ones that may cause delamination.

Embodiments of the inventions are described in more detail with reference to the accompanying schematic drawings that are listed below
- Fig. 1: depicts a first embodiment of a molding arrangement;
- Fig. 2: depicts a second embodiment of a molding arrangement;
- Fig. 3: depicts a third embodiment of a molding arrangement;
- Fig. 4: depicts a fourth embodiment of a molding arrangement;
- Fig. 5: depicts a fifth embodiment of a molding arrangement;
- Fig. 6: depicts a first embodiment of a feedthrough arrangement for use in a molding arrangement;
- Fig. 7: depicts a second embodiment of a feedthrough arrangement for use in a molding arrangement;
- Fig. 8: depicts a third embodiment of a feedthrough arrangement for use in a molding arrangement;
- Fig. 9: depicts the feedthrough arrangement of Fig. 8 with the upper mold removed; and
- Fig. 10: depicts an embodiment of the second invention for monitoring a fiber composite manufacturing process.

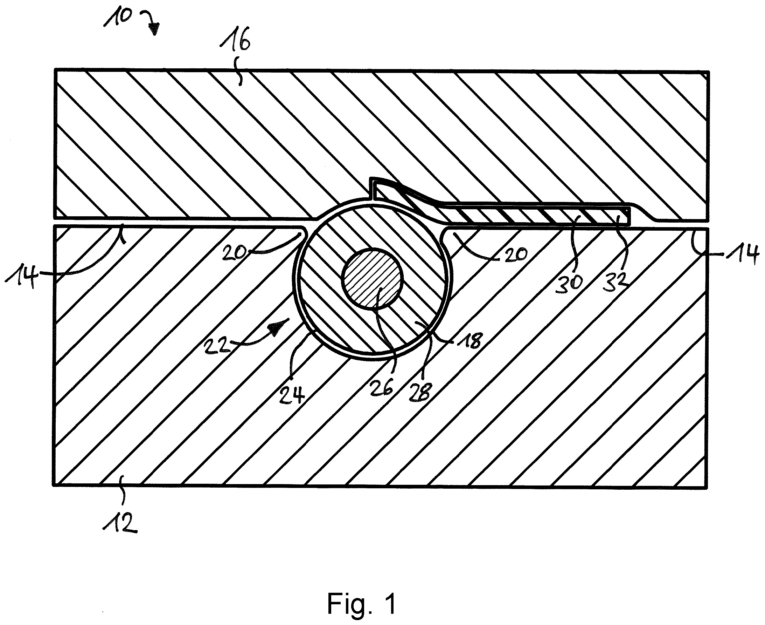

Referring to Fig. 1, a molding arrangement 10 is depicted that may be used in a molding apparatus during a molding process, such as RTM. The molding arrangement 10 comprises a mold 12. The mold 12 includes a molding surface 14. The molding surface 14 faces a preform 16 that is to be infused with resin.

The mold 12 includes an accommodation groove 18 that is formed within the mold 12. The accommodation groove 18 includes at least one retaining feature 20. The retaining feature 20 is configured for retaining a sensor apparatus 22 and keeping it from moving out of the accommodation groove 18 due to its own weight.

The molding arrangement 10 comprises the sensor apparatus 22. The sensor apparatus 22 is arranged within the accommodation groove 18. The sensor apparatus 22 protrudes from the accommodation groove 18 toward the preform 16. The sensor apparatus 22 is partially integrated into the mold 12. Here, more than half of the sensor apparatus 22 is below the molding surface 14, while a smaller part protrudes. The sensor apparatus 22 includes a sensor element 24. The sensor element 24 has a signal wire 26 that is embedded in a dielectric 28. The dielectric 28 may engage the preform. The sensor element 24 has a circular cross section.

In a variant, the sensor apparatus 22 includes a retaining member 30, e.g. an adhesive tape. Furthermore, the sensor apparatus 22 may include a sealing member 32 that prevents race tracking. The sealing member 32 may also be formed by adhesive tape.

The molding arrangement 10 further includes a control unit (not shown). The control unit can be implemented using a microcontroller, computer software or control logic circuitry. The control unit is operatively coupled to the sensor apparatus 22 and may perform a method as known from EP 3 035 041 A1 and EP 3 035 042 A2, the disclosure of which is incorporated herein by reference insofar as it pertains to the material parameter detection method.

With this, the control unit energizes using time domain reflectometry the signal wire 26 that generates an electric field between the signal wire 26 and the mold 12, which may serve as ground. The electric field interacts with the preform 16 and during the molding process with the resin coming into the preform 16. The resin changes the dielectric environment close to the sensor element 24 so that also the electric field is changing. In other words there is again a dielectric discontinuity at the position of the cure or flow front. As a result, the curing and flowing of the resin can be detected by the control unit.

Furthermore, other embodiments of the molding arrangement 10 are described only insofar as they differ from the previously described embodiment.

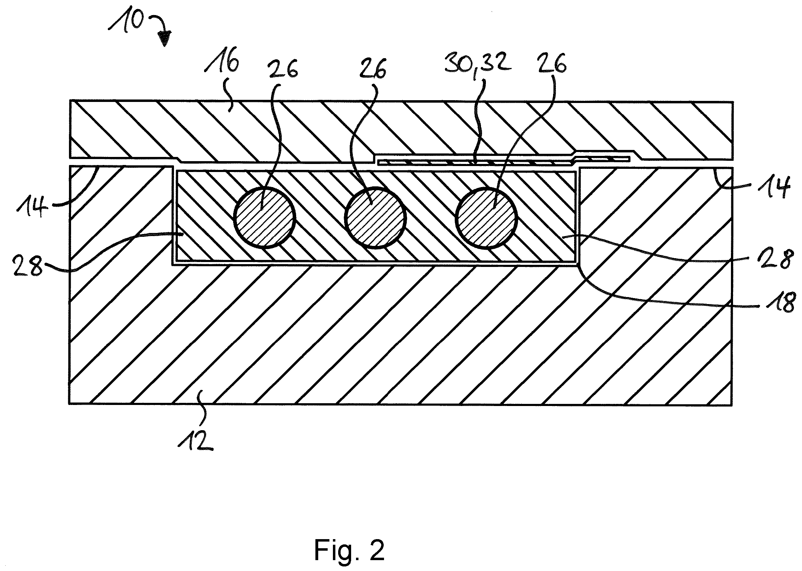

Referring to Fig. 2, the molding arrangement 10 comprises a different cross section of the sensor element 24 that is rectangular shape. The sensor element 24 includes three signal wires 26 that are embedded in the rectangular shaped dielectric 28. During measurement, the control unit may apply positive potential to the central wire and ground potential to the adjacent wires, so that an electric field protruding into the preform is generated. The general measurement principle is the same. It is also possible, that the control unit applies positive and negative potential to the outer wires and ground potential to the central wire, thereby allowing for a differential measurement.

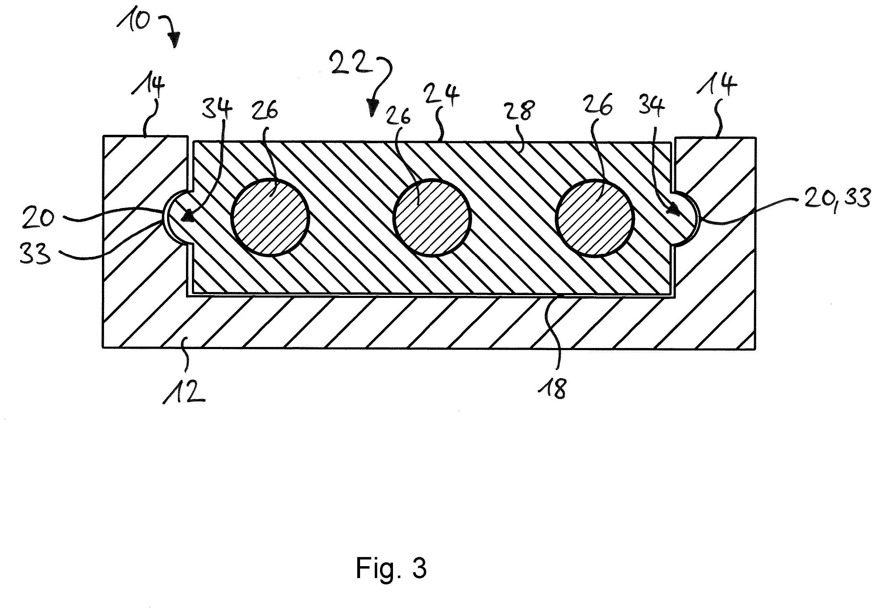

Referring to Fig. 3, the accommodation groove 18 includes a mounting recess 33 forming the retaining member 30. Accordingly, the sensor apparatus 22 has a nib 34 matching the mounting recess 33. The nib 34 may be integrally formed to the sensor element 24 from the dielectric 28.

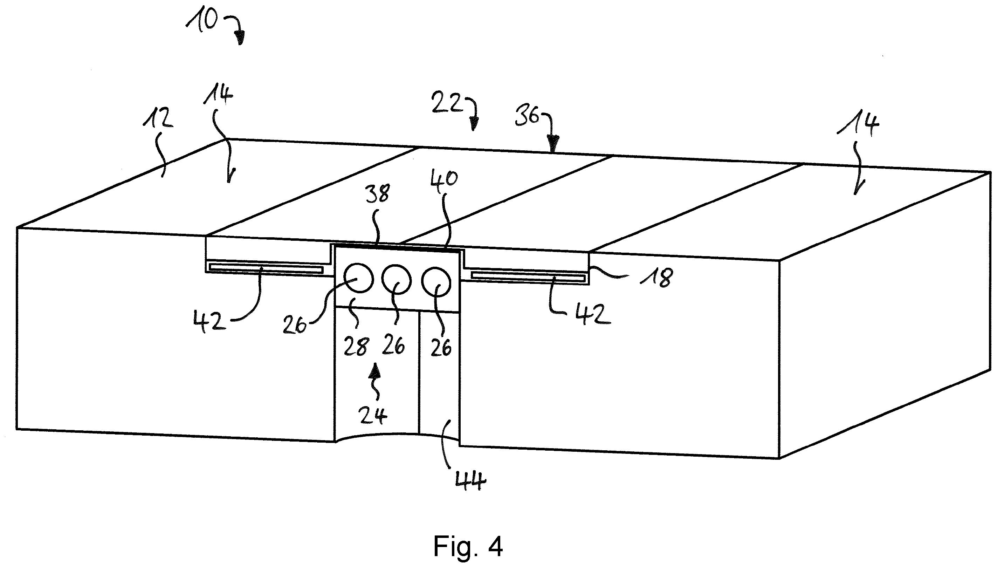

Referring to Fig. 4, the sensor apparatus 22 includes a lid member 36. The lid member 36 is made of metal, preferably the same metal as the mold 12 or the molding surface 14. The lid member 36 includes a second accommodation groove 38. The sensor element 24 is attached to the lid member 36 and arranged within the second accommodation groove 38. The lid member 36 also has a thinned portion 40 that is adjacent to the second accommodation groove 38. The thickness of the thinned portion 40 is chosen so as to allow interaction of an electric field generated by the signal wires 26 with the preform 16 and the cure and flow front. The sensor apparatus 22 further includes a lid sealing member 42 that is arranged between the lid member 36 and the mold 12. The mold 12 also includes a feedthrough recess 44 for connecting the sensor element 24 to the control unit via a cable.

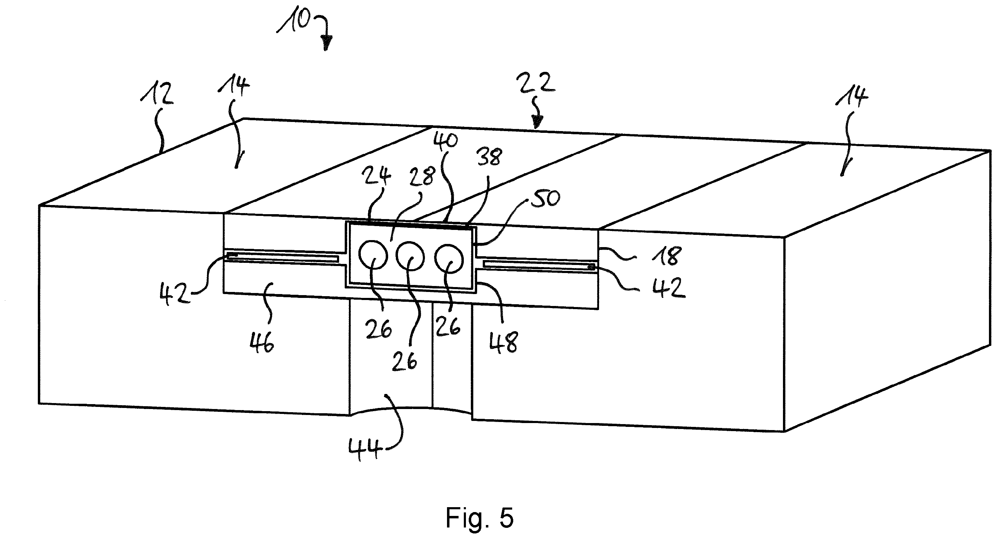

Referring to Fig. 5, the sensor apparatus 22 differs from that of Fig. 4 in that the sensor apparatus further comprises a core member 46. The core member 46 includes a third accommodation groove 48. Both the second and third accommodation groove 38, 48 form an accommodation channel 50 in which the sensor element 24 is arranged. The lid sealing member 42 is arranged between the lid member 36 and the core member 46.

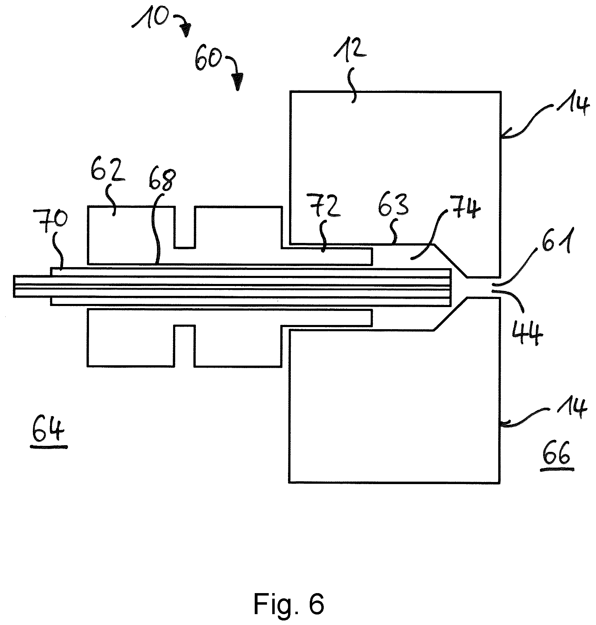

Referring to Fig. 6, a feedthrough arrangement 60 is described that may be used in the mold arrangement 10. The feedthrough arrangement 60 comprises the mold 12. The mold 12 has a through hole 61 and a threaded mounting hole 63. The feedthrough arrangement 60 further comprises a feedthrough element 62. The feedthrough element 62 is configured for feeding a connector from an ambient environment 64 through the mold 12 into a molding chamber 66, so as to connect to the sensor element 24. The feedthrough element 62 includes a feedthrough passage 68 for a sensor connector 70. The feedthrough element 62 comprises a threaded portion 72. The threaded portion 72 has a conical thread. The threaded portion 72 matches the threaded mounting hole 63. The feedthrough element 62 defines a resin chamber 74 into which resin may ingress without reaching the threaded portion 72.

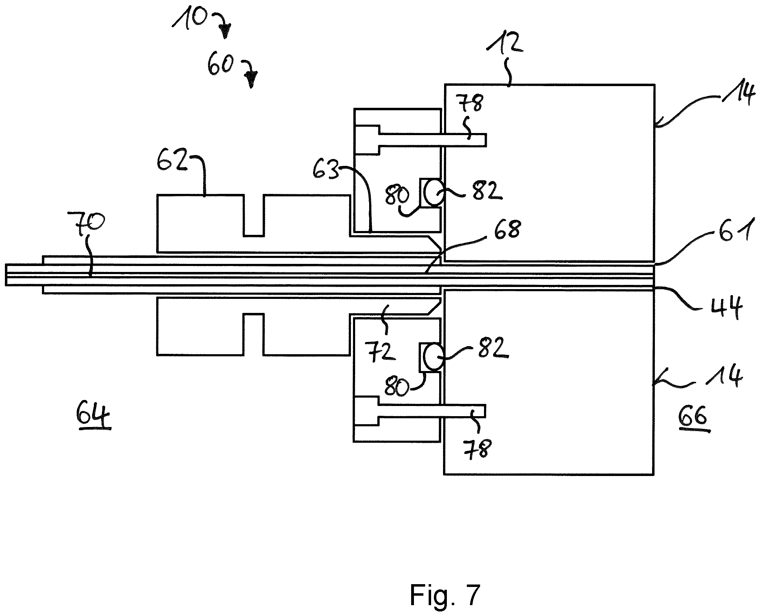

Referring to Fig. 7, the feedthrough arrangement 60 comprises a mounting plate 76. In this embodiment, the mounting plate 76 has the threaded mounting hole 63 formed therein. The mounting plate 76 is fixed to the mold 12 using threaded fasteners 78. The mounting plate 76 makes planar contact with the mold 12. The mounting plate 76 includes a sealing groove 80. The sealing groove 80 is annularly shaped. The sealing groove 80 has arranged therein a sealing element 82. The sealing element 82 is pressed against the mold 12, when the mounting plate 76 is in the mounted state.

During the molding process, the resin is prevented from reaching the threaded fasteners 78. The resin may still form a thin layer between the mounting plate 76 and the mold 12. However, due to the planar contact and possible mechanical advantage, the mounting plate 76 can be easily removed.

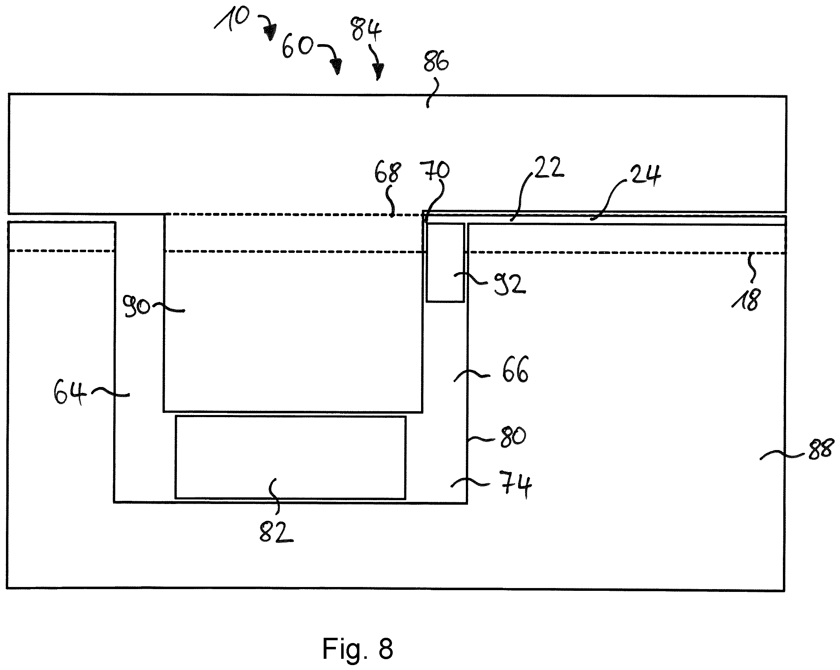

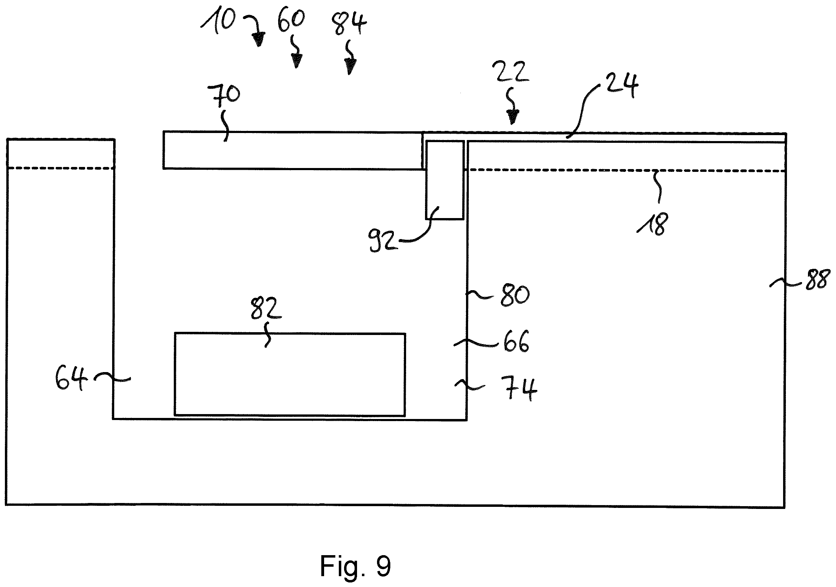

Referring to Fig. 8 and Fig. 9, the feedthrough arrangement 60 includes a multi-part mold 84, having a first mold portion 86 and a second mold portion 88. The first mold portion 86 includes a mold protrusion 90. The mold protrusion 90 has formed therein the feedthrough passage 68. The sensor connector 70 is fixed in the feedthrough passage 68. The sensor connector 70 protrudes from the feedthrough passage 68 so as to be capable of operatively coupling to the sensor apparatus 22. The sensor apparatus 22 is arranged in its accommodating groove 18 formed in the second mold portion 88. The second mold portion 88 further includes the sealing groove 80. The sealing groove 80 is formed to match the mold protrusion 88. The sealing groove 80 includes the sealing element 82 at its bottom. The resin chamber 74 is defined between the first and second mold portions 86, 88 on the side of the molding chamber 66. The feedthrough arrangement 60 further comprises a locking mechanism 92 that is arranged in the resin chamber 66 adjacent to the sensor connector 70 and the sensor apparatus 22. When the multi-part mold 84 is closed, the locking mechanism 92 locks the sensor connector 70 and the sensor apparatus 22 together. When the multi-part mold 84 is opened, the locking mechanism 92 disengages and the sensor connector 70 disconnects from the sensor apparatus 22. The locking mechanism 92 may be a clip-type locking mechanism.

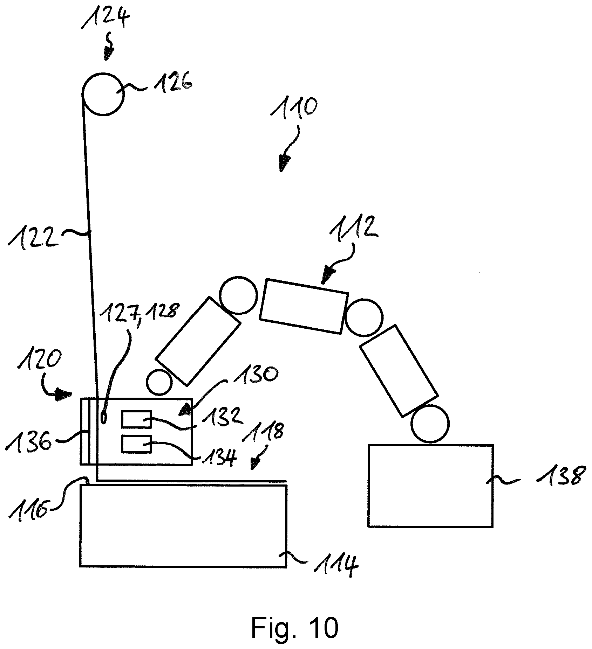

In the following, the second invention is described in more detail with reference to Fig. 10, which depicts a manufacturing apparatus 110. The manufacturing apparatus 110 is configured for automated fiber placement. The manufacturing apparatus 110 comprises an automated fiber placer 112 and a tool 114 with a tool surface 116 on which a fiber composite part 118 is to be manufactured.

The automated fiber placer 112 is configured in a usual manner. The automated fiber placer 112 includes a fiber material placement head 120. The fiber material placement head 120 is configured for automatically placing fiber material 122, which is preferably a prepreg material, onto the tool surface 116 or a surface of the fiber composite part under manufacture.

The manufacturing apparatus 110 includes a fiber source 124. The fiber source is typically integrated in the automated fiber placer 112, specifically the fiber material placement head 120. For purposes of clarity it is depicted separate therefrom.

The fiber source 124 includes at least one fiber storage 126, preferably in the form of a spool. The fiber source 124 includes the fiber material 122, preferably in the form of prepreg tow. Adjacent layers of the fiber material 122 are separated from each other using a separation member, preferably in the form of thin foil or - sometimes colored - tape.

During the manufacturing process, it may happen that a piece of separation member residue 127 or foreign object 128 sticks to the fiber material 122.

The manufacturing apparatus 110 further includes a sensor apparatus 130. The sensor apparatus 130 is configured for detecting the separation member residue 127 or the foreign object 128. The sensor apparatus 130 may include a computer vision algorithm that it suitable for object detection. Such algorithms are known per se.

The sensor apparatus 130 may include a scanning sensor 132, preferably in the form of an imaging sensor. Furthermore, the sensor apparatus 130 may include a light source 134 which is arranged to illuminate the fiber material 122. The sensor apparatus 130 may also include a non-reflective background 136, wherein the fiber material 122 is arranged between the scanning sensor 132 and the non-reflective background 136.

The sensor apparatus 130 is arranged such that a scan of the fiber material 122 takes place before it is placed on the tool surface 116 or the surface of the composite part 118.

If the sensor apparatus 130 detects the presence of separation member residue 126 or the foreign object 128, respectively, an interruption signal is generated and processed by a control unit 138.

The control unit 138 is generally configured to control the manufacturing process performed by the manufacturing apparatus 110. Upon receipt of the interruption signal, the control unit 138 may interrupt the manufacturing process and allow for a removal of the separation member residue 126 or the foreign object 128. This may be performed manually. Furthermore, it is possible for the control unit 138 to make a record of the interruption.

After the separation member residue 127 or the foreign object 128 are removed, the control unit 138 may continue with the manufacturing process.

### List of reference signs:

- 10: molding arrangement
- 12: mold
- 14: molding surface
- 16: preform
- 18: accommodation groove
- 20: retaining feature
- 22: sensor apparatus
- 24: sensor element
- 26: signal wire
- 28: dielectric
- 30: retaining member
- 32: sealing member
- 33: mounting recess
- 34: nib
- 36: lid member
- 38: second accommodation groove
- 40: thinned portion
- 42: lid sealing member
- 44: feedthrough recess
- 46: core member
- 48: third accommodation groove
- 50: accommodation channel
- 60: feedthrough arrangement
- 61: through hole
- 62: feedthrough element
- 63: threaded mounting hole
- 64: ambient environment
- 66: molding chamber
- 68: feedthrough passage
- 70: sensor connector
- 72: threaded portion
- 74: resin chamber
- 76: mounting plate
- 78: threaded fasteners
- 80: sealing groove
- 82: sealing element
- 84: multi-part mold
- 86: first mold portion
- 88: second mold portion
- 90: mold protrusion
- 92: locking mechanism

- 110: manufacturing apparatus
- 112: automated fiber placer
- 114: tool
- 116: tool surface
- 118: fiber composite part
- 120: fiber material placement head
- 122: fiber material
- 124: fiber source
- 126: fiber storage
- 127: separation member residue
- 128: foreign object
- 130: sensor apparatus
- 132: scanning sensor
- 134: light source
- 136: non-reflective background
- 138: control unit

## Claims

1. A system for detecting a cure and/or flow front in a molding apparatus, the system comprising:
- a mold portion having a molding surface (14) that is configured to contact a preform (16) to be infused with resin;
- a sensor apparatus (22) is arranged within the mold portion and includes a sensor element (24) that extends in a longitudinal direction and is arranged to be at least partially below the molding surface (14) into the mold portion such that the sensor element (24) is capable of interaction with resin and/or the preform (16); and
- a control unit that is configured to control the sensor element (24), so as to detect a cure and/or flow front based on a change in the interaction due to the flowing or curing of resin.

2. The system according to claim 1, wherein the sensor element (24) is configured for generating an electric field that is capable of interacting with the resin and/or the preform (16), wherein the control unit is configured to supply to and to receive from the sensor element (24) electrical energy, so as to detect a cure and/or flow front based on a change of the electrical field due to the flowing or curing of resin.

3. The system according to claim 2, wherein the sensor element (24) includes a dielectric (28) and at least one signal wire (26) embedded in the dielectric (28), wherein the signal wire (26) is arranged such that, when the signal wire (26) generates an electrical field, the electrical field is capable of interacting with resin and/or the preform (16).

4. The system according to claim 3, wherein the sensor element (24) includes an odd number of signal wires (26) and the control unit is configured to cause generation of an electrical field between pairs of adjacent signal wires (26).

5. The system according to any of the preceding claims, wherein the mold portion includes an accommodation groove (18) that accommodates and engages the sensor apparatus (22) or the sensor element (24), and the accommodation groove (18) is open towards the preform (16).

6. The system according to claim 5, wherein the accommodation groove (18) includes at least one retaining feature (20) that retains the sensor apparatus (22) or the sensor element (24), in a direction orthogonal to the molding surface (14) and/or the sensor apparatus (22) or the sensor element (24) include at least one retaining member (30) that is detachably fixed to the mold portion and/or the molding surface (14), so as to prevent the sensor apparatus (22) and/or the sensor element (24) from moving out of the accommodation groove (18) under its own weight.

7. The system according to any of the preceding claims, wherein the sensor apparatus (22) comprises a sealing member (32) that is fixed, preferably detachably fixed, to the mold portion and that is arranged to seal the sensor element (24) from the preform in order to prevent resin flow along the longitudinal direction of the sensor apparatus (22).

8. The system according to any of the preceding claims, wherein the sensor apparatus (22) comprises a lid member (36) that includes a second accommodation groove (38) and that is attached to the mold portion or to another member, and the sensor element (24) is accommodated in the second accommodation groove (38).

9. The system according to claim 8, wherein a lid sealing member (42) is arranged between the lid member (36) and the mold (12); and/or the lid member (36) has a thinned portion (40) adjacent to the second accommodation groove (38), the thinned portion (40) being configured so as to allow interaction of the sensor element (24) with resin or the preform (16).

10. The system according to claim 8 or 9, wherein the sensor apparatus (22) further comprises a core member (46) that includes a third accommodation groove (48) and is the another member to which the lid member (36) is attached, and the second and third accommodation grooves (38, 48) form an accommodation channel (50) that accommodates the sensor element (24).

11. The system according to claim 10, wherein the depth of the second accommodation groove (38) is greater than the depth of the third accommodation groove (48) so that the sensor element (24) is closer to the molding surface (14) in an orthogonal direction relative to the molding surface (14).

12. The system according to any of the preceding claims, wherein the mold portion includes a feedthrough recess (44) that is configured to allow feedthrough of a connecting cable to the sensor element (24).

13. A molding apparatus for molding a preform into a finished part by introducing resin into the preform (16), the molding apparatus comprising a system according to any of the preceding claims and a mold (12) that has the mold portion.

14. A molding arrangement (10) occurring during a molding process in a molding apparatus according to claim 13, the molding arrangement (10) comprising a preform (16) or a resin infused preform arranged on the molding surface (14), wherein the control unit controls the sensor element (24) to so as to detect the preform (16) and/or resin in the vicinity of the sensor element (24).

15. A method for detecting a cure and/or flow front in a molding process using a system according to any of the claims 1 to 12, a molding apparatus according to claim 13 or a molding arrangement (10) according to claim 14, the method comprising:
- at least partially arranging a sensor apparatus (22) that has a sensor element (24) that extends in a longitudinal direction within a mold portion and arranging the sensor element (24) to be at least partially below the molding surface (14) into the mold portion such that the sensor element (24) is capable of interacting with resin and/or the preform (16);
- with a control unit controlling the sensor element (24) for detecting a cure and/or flow front based on a change in the interaction due to the flowing or curing of resin.
